# EUROPEAN PATENT SPECIFICATION

(11) **EP 2 407 103 B1**
(45) Date of publication and mention of the grant of the patent: **27.11.2013**
(21) Application number: 10168711.9
(22) Date of filing: 14.07.2010
(51) Int. Cl.: A61B 5/097, A61M 16/08, G01N 1/22

(54) **Fluid connection for reducing a fluid volume in the connection**
Flüssigkeitsverbindung zum Verringern einer Flüssigkeitsmenge in einer Verbindung
Connexion de fluide pour réduire le volume de fluide dans la connexion

(43) Date of publication of application: 18.01.2012
(73) Proprietor: General Electric Company, Schenectady, NY 12345 (US)
(72) Inventor: Weckström, Kurt, 00031, Helsinki (FI)
(74) Representative: Kanerva, Arto

(56) References cited:
- EP-A2- 0 856 327
- US-B1- 6 484 724
- US-B2- 7 500 483

## Description

### BACKGROUND OF THE INVENTION

This disclosure relates generally to a fluid connection for reducing a fluid volume in said connection. Especially the disclosure describes the fluid connection to reduce an extra fluid volume when using tapered connectors. The disclosure relates also to sample tubing connections used in analyzing equipment such as gas analyzing equipment for patient respiratory gas.

In anesthesia or in intensive care, the condition of a patient is often monitored e.g. by analyzing the air exhaled by the patient for its carbon dioxide content. For this reason a small portion of the respiratory gas is delivered to a gas analyzer. The sample is carried along a sampling tube connected in one end often to a respiratory tube adapter and the other end to the gas analyzer. This sampling tube is typically disposable and must have some kind of reliable and tight but simple and cheap connectors. Almost all pneumatic connectors in the respiratory system have tapered conical contact surfaces. Such connectors are simple, easy to connect and cheap to make and they still provide an airtight and reliable connection. The connection such as a well-known fitting called Luer-Lok, a registered trademark of Becton Dickinson of Franklin Lakes, NJ USA, has been in general use for gas sampling but also other similar connectors with differing dimensions can be used. The tapered portion of the connector is normally conical with straight cross section sides because it gives a reliable and tight connection using a large contact area. The tapered portion could in principle also have curved cross section sides or one tapered connector in combination with a suitably designed semi-rigid counterpart. The contact surface responsible for the tightness is always on the tapered portion of the connector. Other possibilities would be cylindrical connectors with either axial or radial gaskets but they are more complicated and expensive and, consequently, not suitable as disposable components. Such connectors are typically used e.g. in pressurized gas lines or gas lines of more permanent nature. With this design it would be easier to avoid dead space in the connection bore since they are allowed to touch axially, longitudinally, while still remaining airtight.

A gas analyzer designed to measure respiratory gas in real time has to be fast enough to resolve changes in the gas content. This is especially true for carbon dioxide, which varies from close to zero in the inspiratory phase to about 5 % in the expiratory phase of the breathing cycle. It is then very important to streamline the complete gas sampling system. Many portions of the system with slowed down response can easily add up to unacceptable performance of the gas analyzer. The reason for an increased rise time of e.g. carbon dioxide is often an extra fluid volume, a dead space in the pneumatic line, where the gas flow is slowed down. The tapered conical connector is susceptible to such dead space, especially if the inner dimensions are significantly larger than those of the bore or sampling line itself. The inherent construction of the conical connector is such that dead space always is introduced and the amount is critically dependent on the tolerance of the conical dimensions. The connectors must allow for axial or longitudinal play in order to avoid the situation of touching axially because then air leak is likely to occur. Therefore, the tolerances always define an axial extra fluid volume in the connection to ensure tightness at the conical surfaces.

Minimal dead space is essential also in gas or liquid chromatography. An attempt to make connections with capillaries is described in US 6,969,095. The female part of the connection is slightly tapered in order to accept the cylindrical capillary tube and make a tight press-fit. This connector fitting is specially designed for conditions encountered in liquid or gas chromatography and is not intended for repeatedly made reliable connections like in gas analyzers. Robustness inevitably adds dead space to the bore of the connection.

In neonatal main ventilation circuit's extra fluid volume has to be as small as possible. There are different solutions to this problem. The connections are also conically tapered even if the dimensions are much larger than what would be used for a gas sampling system. In one solution there is a sliding internal passage filling the dead space and in another solution a compressible member is used to exclude the extra fluid volume. However, especially for small and disposable connectors like those used in sampling lines of gas analyzers such moving or compressible features would be difficult to implement and would add to the expenses of a disposable accessory.

US 7,500,483 discloses a prior art airway connection between an endotracheal tube adapter and a ventilation tube connector leaving therebetween a void volume. Part of this volume has been filled to eliminate an abrupt diameter change by means of an inner wall of the connector protruding from the connector body.

### BRIEF DESCRIPTION OF THE INVENTION

The above-mentioned shortcomings, disadvantages and problems are addressed herein which will be understood by reading and understanding the following specification.

In an embodiment, a fluid connection for reducing a fluid volume in the connection includes a first connector having a first body encircling a first bore allowing a fluid flow along the first bore, the first body having a first sealing surface The fluid connection also includes a second connector having a second body encircling a second bore allowing a fluid flow along the second bore, the second body having a second sealing surface capable to form a tight connection with the first sealing surface when both the first connector and the second connector are mated. The fluid connection also includes a volume between the first connector and the second connector when mated and a protrusion in of one of the first body encircling the first bore and the second body encircling the second bore and which protrusion is encircling one of the first bore and the second bore. The fluid connection further includes a cavity in a remaining one of the first body and the second body and which cavity is configured to receive the protrusion when maiting the first connector and the second connector separating the volume into a first volume and a second volume which is at a distance from the first volume.

In another embodiment, a fluid connection for reducing a fluid volume in the connection includes a first connector having a first body encircling a first bore allowing a fluid flow along the first bore, the first body having a first sealing surface The fluid connection also includes a second connector having a second body encircling a second bore allowing a fluid flow along the second bore, the second body having a second sealing surface capable to form a tight connection with the first sealing surface when both the first connector and the second connector are mated. The fluid connection also includes a volume between the first connector and the second connector when mated, a cross-sectional area of the volume being larger compared to a corresponding cross-sectional area of one of the first bore and the second bore in fluid communication with the volume, and a protrusion in of one of the first body encircling the first bore and the second body encircling the second bore and which protrusion is encircling one of the first bore and the second bore. The fluid connection further includes a cavity in a remaining one of the first body and the second body and which cavity is configured to receive the protrusion when maiting the first connector and the second connector separating the volume into a first volume and a second volume which is at a distance from the first volume.

In yet another embodiment a fluid connection for reducing a fluid volume in the connection includes a first connector having a first body encircling a first bore allowing a fluid flow along the first bore, the first body having a first sealing surface The fluid connection also includes a second connector having a second body encircling a second bore allowing a fluid flow along the second bore, the second body having a second sealing surface capable to form a tight connection with the first sealing surface when both the first connector and the second connector are mated. The fluid connection also includes a volume between the first connector and the second connector when mated, a cross-sectional area of the volume being larger compared to a corresponding cross-sectional area of one of the first bore and the second bore in fluid communication with the volume, and a protrusion in of one of the first body encircling the first bore and the second body encircling the second bore and which protrusion is encircling one of the first bore and the second bore. The fluid connection further includes a cavity in a remaining one of the first body and the second body and which cavity is configured to receive the protrusion when maiting the first connector and the second connector separating the volume into a first volume and a second volume which is at a distance from the first volume. The first volume and the second volume allows a minor gas exchange with each other by means of an exchange channel between the protrusion and the cavity when the first connector and the second connector are mated.

Various other features, objects, and advantages of the invention will be made apparent to those skilled in art from the accompanying drawings and detailed description thereof.

### BRIEF DESCRIPTION OF THE DRAWINGS

Fig. **1** illustrates a schematic perspective view of a typical monitoring situation with intubated subject and with various fluid connections;

Fig. 2 illustrates as prior art a conically tapered fluid connection;

Fig. **3** shows in a graph how an extra fluid volume of the fluid connection contributes to the response time of a gas concentration measurement;

Fig. **4** depicts one embodiment of a conically tapered fluid connection to reduce the influence of the extra fluid volume in the fluid connection;

Fig. **5** depicts another embodiment of a conically tapered fluid connection to reduce the influence of the extra volume in the fluid connection;

Fig. **6** depicts a third embodiment of a conically tapered fluid connection to reduce the influence of the extra volume in the fluid connection;

Fig. **7** depicts a fourth embodiment of a conically tapered fluid connection to reduce the influence of the extra volume in the fluid connection;

Fig. **8** depicts a fifth embodiment of a tapered fluid connection to reduce the influence of the extra volume in the fluid connection;

Fig. **9** depicts a sixth embodiment of a tapered fluid connection to reduce the influence of the extra volume in the fluid connection; and

Fig. **10** depicts an airway adapter with fluid connection to an intubation tube for neonatal respiratory care in accordance with an embodiment.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

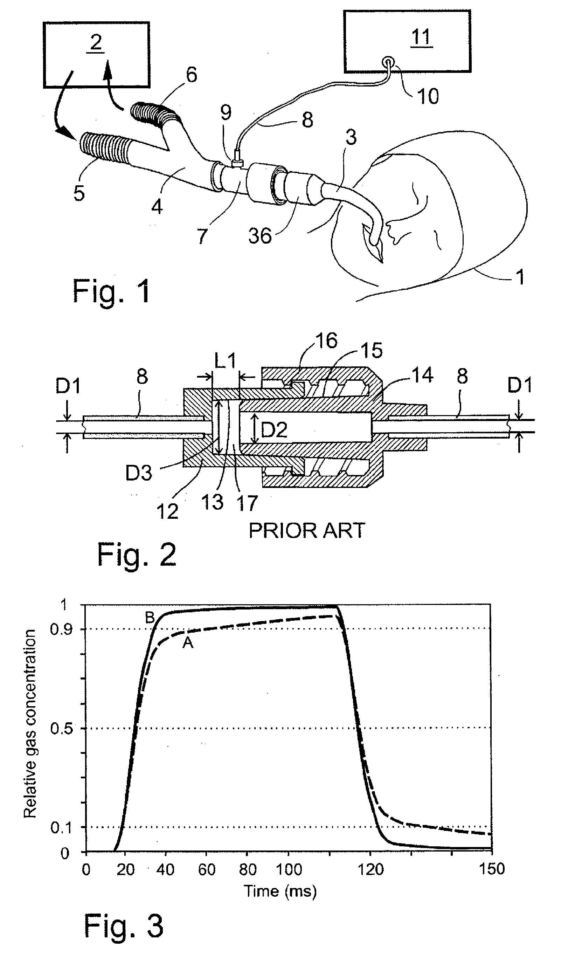

A fluid connection such as a gas connection for reducing an extra fluid volume such as a dead space in this fluid connection is described. This kind of extra fluid volume is especially inherent in a tapered connection, which are commonly used in patient respiratory circuits. Such a respiratory circuit with a medical gas analyzer is shown in Fig. 1. A patient I is connected to a ventilator 2 using an intubation tube 3, a Y-piece 4, an inspiratory limb 5 and an expiratory limb 6. In a respiratory circuit an airway adapter 7 for fluid sampling is usually included. All breathing tube connectors in this breathing circuit are typically conically tapered but the extra volume is critical especially for neonatal use. The reason for the extra volume problems with neonatal ventilation is that the tubing dimensions are designed for adult or pediatric use. Extra volume in a breathing circuit means that the gas exchange in the neonatal lungs are impaired because of inpiratory and expiratory gas mixing. This embodiment could also improve the respiratory gas connections. Also the fluid sampling line 8 is connected using tapered connection fittings in the adapter end 9 and at the input 10 to the gas analyzer 11. These connectors are usually identical and could be conically tapered like well-known fluid connections on the market or otherwise tapered as described further on.

In Fig.2 a prior art fluid connection is depicted. It consists of a female connector 12 with conically tapered inner surface 13 and a male connector 14 with a corresponding conically tapered outer surface 15. Additionally, there is a threaded attachment 16 to secure the connection. This threaded attachment is not always present because a tapered connection easily remains in position and airtight by friction of its surfaces. At least one end of the connection fitting is normally attached to the sampling line 8 with an inner diameter D1. For gas sampling this diameter is normally about 1 mm. Inside the male connector this diameter is often widened to about D2 = 2.5 mm, obviously for manufacturing reasons. This extra volume certainly adds to the extra volume of the connection but can easily be eliminated by reducing the diameter or by extending the sampling line 8 closer to the connector tip. According to the specifications of a well-known connector the inner diameter D3 of the female connector at its bottom end is about 3.8 mm. Since the connectors cannot be allowed to touch axially a clearance and tolerance with length L1 is possible to calculate using the given tolerances of the 6% or about 3.4 degrees tapered portion. This length L1 can vary between a minimum of 1.0 mm and about 2.8 mm. It also defines the extra volume 17 such as an extra space inside the connection. Especially because of its diameter D3, which is almost four times the diameter D1 of the sampling line 8, the extra volume will have an influence on the response time of the gas analyzer 11. The reason for this is that part of the gas flow fills up this volume, which then functions as a reservoir for gas composition from a time period previous to the on-going flow time.

For a more or less stable gas composition the extra volume may not have any major impact on the measurement but for fast changes in gas composition the situation is different, especially when using a fast gas analyzer. This is shown in the graph of Fig. 3. The dashed line A represents the measurement of a typical gas concentration value rising quickly from about zero to a maximum relative value of 1 as a function of time in milliseconds. This could be a graph of the contribution from the tapered connection according to prior art to the response time of exhaled carbon dioxide from a patient as measured by the gas analyzer 11, rising from zero to about 5 % of volume. The rise time is defined as the time between 10% and 90% of the maximum measured value, in this case 1. As can be seen, the curve reaches its final value very slowly, leaving a tail of previous gas, e.g. partly the inhaled gas, free of carbon dioxide. This tail often starts already before the signal has reached its 90% value with a considerable increase in the rise time value as consequence. The same applies to the fall time from the maximum of 1 to zero. The rise time of curve A is about 40 ms when it is supposed to be only about 10 ms. If the pneumatic system of the gas analyzer 11 otherwise is optimized this tail could reduce accuracy even beyond specification. Several similar connections in the sampling line would, of course, further worsen the situation. Needless to say, the same applies even more pronounced if the tapered connection would have larger dimensions than what was given above. Note that the curve only shows the contribution from a tapered connection, not the final breathing curve, the capnogram, of a patient. The short expiratory time period of about 100 ms is used only for illustrating purposes.

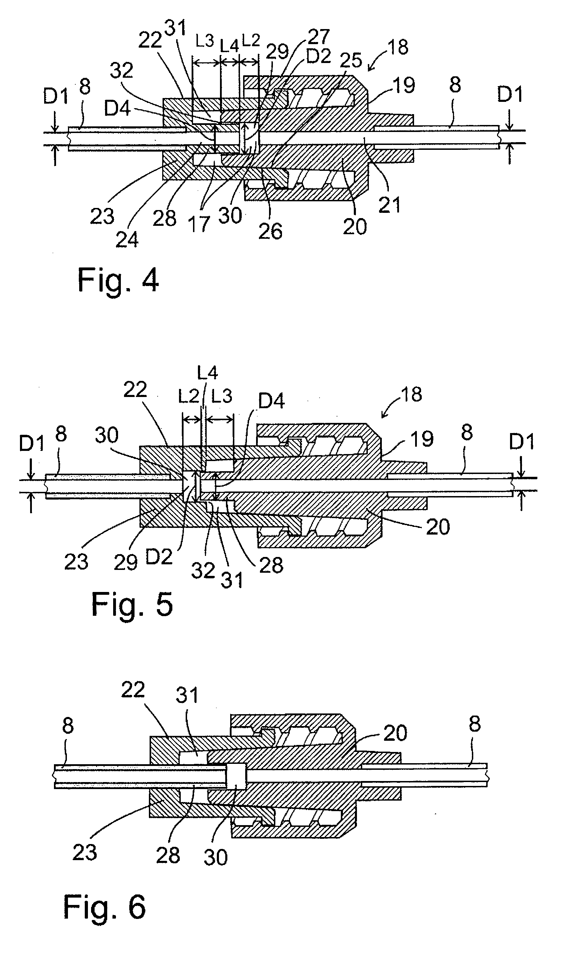

In accordance with an embodiment the extra volume of the fluid connection can be reduced to an acceptable level using a rigid or semi-rigid structure, which may be for example cylindrical and which is able to allow for the considerable axial play of a tapered connection. Such embodiment is depicted in Fig. 4. For simplicity, the same type of the fluid connection is considered as in Fig. 2. The fluid connection 18 comprises both a first connector 19 having a first body 20 encircling a first bore 21 and a second connector 22 having a second body 23 encircling a second bore 24. Said first and second connectors are preferably detachable from each other. Said first body 20 comprises also a first sealing surface 25 as well as said second body 23 comprises a second sealing surface 26 capable to form a tight connection with said first sealing surface 25 when both said first connector 19 and said second connector 22 are mated. Also the embodiment may be equipped with a threaded attachment 27 to secure the dependable connection between said first connector 19 and said second connector 22. Either said first body 20 encircling said first bore 21 or said second body 23 encircling said second bore 24 is equipped with a protrusion 28 and which protrusion is encircling one said first bore 21 and said second bore 24. A cavity 29 is with the remaining one of said first body and said second body, which remaining one is without said protrusion 28. In Figure 4 embodiment said protrusion 28 is in said second body 23 and said cavity 29 is in said first body 20.

An internal diameter D2 of said cavity 29 has been reduced to a diameter close to the diameter D1 of the sampling line 8, which is operationally connected to said first bore 21 as shown in Figure 4. Also the first bore's diameter is preferably substantially same or identical with the diameter D1 of the sampling line 8. Further said second bore's 24 diameter is preferably substantially same or identical with the diameter D1 of the sampling line 8. Said protrusion may be cylindrical having an outer diameter D4 and an inner diameter D1, which inner diameter is substantially same or identical with that of the sampling line 8. The outer diameter D4 of the protrusion 28 is dimensioned in such a way that it can slide into said cavity 29 with diameter D2 in the first connector 19 without the need to form an airtight connection. When mating said first connector and said second connector, in which case said cavity 29 is receiving said protrusion 28, the extra volume 17 with a larger cross-sectional area compared to corresponding cross-sectional area of one of said first bore and said second bore, which extra volume locating between said first connector 19 and said second connector 22 is separated into two different volumes, which are a first volume 30 such as a first space and a second volume 31 such as a second space which is at a distance from said first volume. Also when mated these first and second volumes exist.

The difference in diameter, D2-D4, should be small enough to allow only a minor gas exchange such as a diffusion between said first volume 30 and said second volume 31 and to only slowly enter the sampling flow through an exchange channel 32, which may be narrow and for example annular, joining said first volume and said second volume. The internal diameter D2 of said cavity 29 is typically identical with the diameter of said first volume 30 and this same applies to a cross-sectional area of said first volume and said cavity which are typically identical. Said second volume's diameter including said protrusion's 28 diameter D4 is in this embodiment longer than said first volume's diameter. This also means that a cross-sectional area of said second volume including said protrusion 28 is wider than a cross-sectional area of said first volume. Further the cross-sectional area of said first volume 30 is wider than a cross-sectional area of one of said first bore 21 and said second bore 24 having the diameter D1. The cross-sectional area discussed herein is considered to be perpendicular to a main direction of the flow in said first and second bores.

In the embodiment of Figure 4 said first volume 30 locates between said first bore 21 and said second bore 24 allowing the fluid flow through this first volume. The second volume may encircle said protrusion 28 when said first body 20 and said second body 23 are mated. The maximum length L2 of the first volume 30 having a cross sectional area with diameter D2 can be smaller than a length L3 of said second volume 31, but must be able to allow for the axial clearance, which in this example is 2.8 mm - 1.0 mm = 1.8 mm. This minimum value of L2 with diameter D2 is small enough to leave the gas measurement almost intact. The result is shown in curve B of Fig. 3. A small tail is still to be seen, but it has no noticeable influence on the clinical measurement. The knee point of the curve B favorably occurs at about 97% of the maximum concentration value when it was only about 87% for curve A of the prior art. It is advisable to have the knee point of the rising curve at > 95% of the maximum when measuring a step change in concentration at the rising edge. At the falling edge the corresponding value would be < 5% or favorably < 3% of the maximum concentration.

The height of the annular exchange channel 32 depends on the allowable gas exchange between said first volume 30 and said second volume 31. The gas exchange or diffusion time depends on the type of gas, the partial pressure difference of this gas and cross section and length of the exchange channel 32. A simple calculation shows that the dimensions of the exchange channel 32 are not very critical because the gas exchange is a very slow process compared to the time scale of concentration change in the sample flow, compare to the time scale of milliseconds in Fig.3. The difference in diameter, D2 - D4, could well be 0.4 mm, which means 0.2 mm for the height of the exchange channel 32. This is a suitable manufacturing tolerance for both small and large connectors but in general terms the ratio between the height of the exchange channel 32 and its diameter, the mean value of D2 and D4, should favorably be less than 0.1. The length L4 of the exchange channel 32 does not have to be more than about 0.5 mm in the example under study. Another mechanism partly responsible for the gas exchange through the exchange channel 32 between the first volume 30 and the second volume 31 is a sudden pressure change resulting in a breathing mechanism. A third mechanism would be that of an ejector due to venturi effect from the net flow in the first bore 21 and second bore 24. However, the dimensions of the exchange channel 32 given above are such that the effect of the said second volume 31 is reduced to an acceptable level despite all these contributions. The advantage of the first connector 19 in the embodiment of Fig. 4 is that it can be used as a standard connector to mate with a prior art counterpart without said protrusion 28. The rise time will be longer but the connection is airtight.

In general terms, the length of the protrusion 28, L3 + L4 should be about the maximum value within the tolerances of L3 with the addition of the minimum acceptable value of the L4, the exchange channel 32. In the example above this would be L3 + L4 = 2.8 mm + 0.5 mm = 3.3.mm. Observe that if L3 has its minimum value L3 = 1 mm, then L4 will be 2.3 mm and the exchange channel 32 will separate said second volume 31 very efficiently. The depth of the corresponding cavity 29 with diameter D2 and length L2 + L4 could be the maximum needed clearance of the connection within specification with the addition of the minimum acceptable value of L4 and a small distance to avoid axial contact. In the example this would be L2 + L4 = 1.8 mm + 0.5 mm + 0.2 mm = 2.5 mm. The small additional distance of 0.2 mm then ensures airtight connection for all connectors within the specification. If L3 would be 1 mm in the example above then said first volume 30 with diameter D2 would be only 0.2 mm long. The method is effective in cases where the diameter of the second volume 31, corresponding to D3 in the prior art connection of Figure 2 is more than twice the diameter D1 of said first bore 21 or said second bore 24 or said gas sampling line 8 and especially if it is more than three times the diameter of said first bore 21 or said second bore 24 or said gas sampling line 8.

Said protrusion 28 in said second body 23 of said second connector 22 of Fig. 4 could also be integrated in said first body 20 of said first connector 19 in which case said cavity 29 with the diameter D2 is in the second body 23 of said second connector 22 as shown in Fig. 5. The outer diameter of such a protrusion 28 would be D4 and the structure would favorably be of the same material as said first body 20 for cost reasons. The first volume 30 and the second volume 31 is similar to that in Fig. 4. In all other respects the connection is functionally identical to the connection in Fig. 4. The advantage of this connection of this embodiment is that the second connector 22 can accept counterpart connectors without the protrusion 28. The rise time will be longer but the connection is airtight.

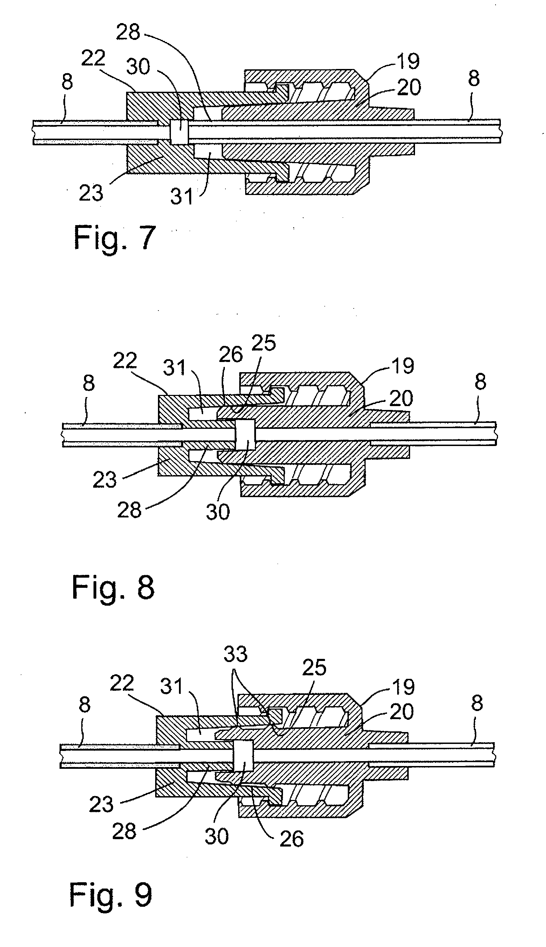

Variations of the two types of connector in Fig. 4 and Fig 5 are shown in Figs 6 and 7, respectively. The difference is that the protrusion 28 is an extension of the fluid sampling line 8 in the second connector 22 according to Fig 6 and the protrusion 28 in the first connector 19 according to Fig.7. In all other respects the connections are functionally identical to those already were described.

The connector embodiments above have had conically tapered contact surfaces. The same type of connection leading to a possible unacceptable extra volume can also result from other types of tapered connection fittings. The connectors depicted in Fig.8 are very similar to those in Fig.4 but only one connector, favorably the second connector 22, is tapered to accept a more or less cylindrical first connector 19. The contact area between said first sealing surface 25 and said second sealing surface 26 responsible for an airtight connection is smaller than for the conically tapered connectors but in most cases also reliable, especially if the material is semi-rigid.

One of said first body 20 and said second body 23 could also have in one of said first sealing surface 25 and said second sealing surface 26 one or several annular ridges 33 to accomplish an airtight connection. In the embodiment of Fig.9 said first sealing surface 25 of said first connector 19 is equipped with said ridges 33,The ridges are in this embodiment of the same material as said first body 20 for economical reasons, but could naturally also be constructed using separate gaskets or O-rings. The second sealing surface 26 of said second body 23 is tapered so the fitting differs in this respect from a cylindrical fitting with radial gaskets and the extra fluid volume is inherent in the construction.

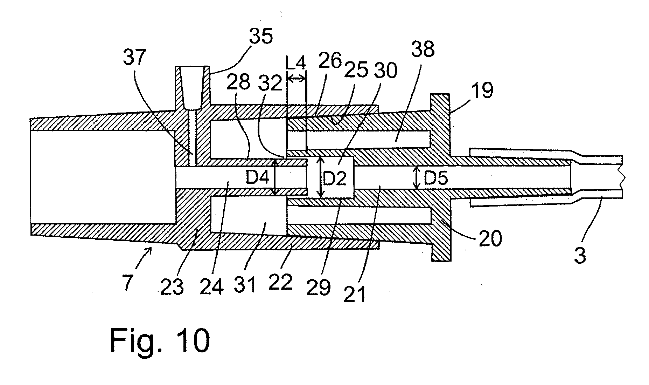

Another type of conically tapered connection is shown in Fig.10. The airway adapter 7 is connected to the intubation tube 3 using a modified conical first connector 19. Naturally this same connection can be used to connect the airway adapter 7 to the ventilator 2. The airway adapter 7 is used to sample the respiratory gas through a small channel 37 and, in this example, via a small tapered connection 35, which could be one of the types described earlier. The adapter is shown in Fig.1 but the internal design of Fig.10 has low extra fluid volume and is to be used for neonates. The second connector 22 of the adapter 7 is provided with a cylindrical or close to cylindrical rigid or semi-rigid protrusion 28 with the second bore 24 such as an internal passage for the respiratory gas. The adapter 7 is further connected to the ventilator 2 as described earlier. The protrusion 28 with outer diameter D4 is favorably of the same material as the airway adapter 7 but could also be of a different material.

The protrusion 28 with a cylindrical structure in Fig.10 is designed to slide into a corresponding cavity 29 with diameter D2 in the first body 20 of said first connector 19. The construction is similar in principle to that in Fig. 4. The only difference is that the dimensions for components in a respiratory circuit are bigger. The diameter of said first sealing surface 25 and said second sealing surface 26 is commonly about 15 mm. The inner diameter D5 of the intubation tube 3 and said second bore 24 as well as the first bore 21 of said first connector 19 can for neonates be as small as 2.5 mm so the extra fluid volume has a major effect on the respiratory gas exchange. The residual first volume 30 in the embodiment of Fig. 10 is very small compared to the original one including besides the first volume also the second volume 31. Again, the protrusion 28 and said cavity 29 do not have to fit closely. The exchange channel 32 only has to be small enough to prevent air exchange fast compared to the respiratory cycle. In practice, its height could be less than 0.5 mm or favorably less than 0.2 mm. The length L4 of the overlapping region could be from a minimum of about 1 mm to a maximum defined by the dimensions and tolerances of the tapered standard connection between said first sealing surface 25 and said second sealing surface 26. Otherwise, the general principles described earlier apply. A space 38 in said first connector 19 is present for reasons related to manufacturing and could also be filled with some material. Both the modified airway adapter 7 and said first connector 19 with its intubation tube 3 can be used with its standard counterpart but the benefit of low extra fluid volume will, of course, then not be fulfilled.

Instead of the connection 35 and sampling channel 37 the adapter 7 could be an adapter for a so called mainstream gas sensor, measuring the infrared absorption directly across the second bore 24. In that case the adapter 7 is provided with two infrared windows according to well-known principles.

Only a few embodiments with different types of tapered connection fittings have been shown above to clarify various embodiments. The common feature is that an unacceptable extra fluid volume inherently is located in the flow channel of the connection. It is to be understood that also other types of connections with this same type of the extra fluid volume problem are possible to improve using the additional rigid or semi-rigid cylindrical structure described.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A fluid connection for reducing a fluid volume in said connection comprising:
a first connector (19) having a first body (20) encircling a first bore (21) allowing a fluid flow along said first bore, said first body having a first sealing surface (25), and which first bore is operationally connected to a sampling line (8) having an inner diameter (D1) or to an intubation tube (3) having an inner diameter (D5);
a second connector (22) having a second body (23) encircling a second bore (24) allowing a fluid flow along said second bore, said second body having a second sealing surface (26) capable to form a tight connection with said first sealing surface (25) when both said first connector (19) and said second connector (22) are mated;
a protrusion (28) in of one of said first body (20) encircling said first bore (21) and said second body (23) encircling said second bore (24) and which protrusion is encircling one of said first bore and said second bore,
a volume (17) between said first connector (19) and said second connector (22) when mated, and
a cavity (29) in a remaining one of said first body and said second body and which cavity is configured to receive said protrusion when mating said first connector and said second connector separating said volume (17) into a first volume (30) and a second volume (31) which is at a distance from said first volume,
**characterized in that** said first and second bore's diameter is substantially same or identical with the diameter (D1) of said sampling line (8) or the diameter (D5) of said intubation tube.

2. The fluid connection according to claim 1, **characterized in that** at least one of said first body (20) with said first sealing surface (25) and said second body (23) with said second sealing surface (26) is equipped with a conical surface forming a fluid tight connection between said first sealing surface and said second sealing surface when mated.

3. The fluid connection according to claim 1, **characterized in that** both said first sealing surface (25) and said second sealing surface (26) are tapered making possible a large enough sealing surface between said first connector (19) and said second connector (22) when mated in which case said first body (20) and said second body (23) are at least partly within each other.

4. The fluid connection according to claim 1, **characterized in that** said second volume (31) is configured to encircle said protrusion (28) when said first body (20) and said second body (23) are mated.

5. The fluid connection according to claim 1, **characterized in that** said first volume (30) and said second volume (31) are configured to allow a minor gas exchange with each other by means of an exchange channel (32) between said protrusion (28) and said cavity (29) when said first connector (19) and said second connector (22) are mated.

6. The fluid connection according to claim 1, **characterized in that** said first volume (30) with a cross-sectional area wider than a corresponding cross-sectional area of one of said first bore (21) and said second bore (24) is configured to locate between said first bore (21) and said second bore (24) allowing the fluid flow through this first volume.

7. The fluid connection according to claim 1, **characterized in that** said cavity (29) is configured to be a part of a remaining one of said first body (20) and said second body (23) being without said protrusion (28).

8. The fluid connection according to claim 1, **characterized in that** said second volume (31) is configured to locate mainly between said protrusion (28) and said second body (23).

9. The fluid connection according to claim 1, **characterized in that** at least one of said first connector (19) and said second connector (22) is operationally connected to a fluid sampling line (8).

10. The fluid connection according to claim 1, **characterized in that** said first connector (19) and said second connector (22) is used to operationally connect an airway adapter to one of an intubation tube (3) and a ventilator (2).

11. The fluid connection according to claim 1, **characterized in that** at least one of said first connector (19) and said second connector (22) is operationally connected to an airway adapter (7).

12. The fluid connection according to claim 1, **characterized in that** said second volume's diameter including said protrusion's (28) diameter is longer than said first volume's diameter.

13. The fluid connection according to claim 1, **characterized in that** a cross-sectional area of said second volume (31) including said protrusion (28) is wider than a cross-sectional area of said first volume (30).

14. The fluid connection according to claim 13, **characterized in that** the cross-sectional area is considered to be perpendicular to a main direction of the flow in said first and second bores.

15. The fluid connection according to claim 1, **characterized in that** said first connector (19) and second connector (22) are detachable from each other.

## Patentansprüche

1. Fluidverbindung zum Verringern eines Fluidvolumens in der Verbindung, umfassend:
einen ersten Verbinder (19) mit einem ein erstes Lumen (21) umgebenden ersten Körper (20) zur Ermöglichung eines Fluidstroms entlang dem ersten Lumen, wobei der erste Körper eine erste Dichtfläche (25) aufweist, und welches erste Lumen funktionell verbunden ist mit einer Probenentnahmeleitung (8), die einen Innendurchmesser (D1) aufweist, oder mit einem Intubationstubus (3), der einen Innendurchmesser (D5) aufweist;
einen zweiten Verbinder (22) mit einem ein zweites Lumen (24) umgebenden zweiten Körper (23) zur Ermöglichung eines Fluidstroms entlang dem zweiten Lumen, wobei der zweite Körper eine zweite Dichtfläche (26) aufweist, die in der Lage ist, eine undurchlässige Verbindung mit der ersten Dichtfläche (25) zu bilden, wenn sowohl der erste Verbinder (19) als auch der zweite Verbinder (22) zusammengepasst sind;
einen Vorsprung (28) in einem von dem das erste Lumen (21) umgebenden ersten Körper (20) und dem das zweite Lumen (24) umgebenden zweiten Körper (23) und welcher Vorsprung eines von dem ersten Lumen und dem zweiten Lumen umgibt,
ein Volumen (17) zwischen dem ersten Verbinder (19) und dem zweiten Verbinder (22), wenn diese zusammengepasst sind, und
einen Hohlraum (29) in einem verbleibenden von dem ersten Körper und dem zweiten Körper und welcher Hohlraum ausgelegt ist, um beim Zusammenpassen des ersten Verbinders und des zweiten Verbinders den Vorsprung aufzunehmen, der das Volumen (17) in ein erstes Volumen (30) und ein zweites Volumen (31) trennt, das sich in einem Abstand von dem ersten Volumen befindet,
**dadurch gekennzeichnet, dass** der Durchmesser des ersten und des zweiten Lumens im Wesentlichen gleich oder identisch mit dem Durchmesser (D1) der Probenentnahmeleitung (8) oder dem Durchmesser (D5) des Intubationstubus ist.

2. Fluidverbindung nach Anspruch 1, **dadurch gekennzeichnet, dass** zumindest einer von dem ersten Körper (20) mit der ersten Dichtfläche (25) und dem zweiten Körper (23) mit der zweiten Dichtfläche (26) ausgestattet ist mit einer konischen Fläche, die eine fluidundurchlässige Verbindung zwischen der ersten Dichtfläche und der zweiten Dichtfläche bildet, wenn diese zusammengepasst sind.

3. Fluidverbindung nach Anspruch 1, **dadurch gekennzeichnet, dass** sich sowohl die erste Dichtfläche (25) als auch die zweite Dichtfläche (26) verjüngt, wobei eine ausreichend große Dichtfläche zwischen dem ersten Verbinder (19) und dem zweiten Verbinder (22) ermöglicht wird, wenn diese zusammengepasst sind, in welchem Fall sich der erste Körper (20) und der zweite Körper (23) zumindest teilweise ineinander befinden.

4. Fluidverbindung nach Anspruch 1, **dadurch gekennzeichnet, dass** das zweite Volumen (31) ausgelegt ist, um den Vorsprung (28) zu umgeben, wenn der erste Körper (20) und der zweite Körper (23) zusammengepasst sind.

5. Fluidverbindung nach Anspruch 1, **dadurch gekennzeichnet, dass** das erste Volumen (30) und das zweite Volumen (31) ausgelegt sind, um einen geringfügigen Gasaustausch miteinander mittels eines Austauschkanals (32) zwischen dem Vorsprung (28) und dem Hohlraum (29) zuzulassen, wenn der erste Verbinder (19) und der zweite Verbinder (22) zusammengepasst sind.

6. Fluidverbindung nach Anspruch 1, **dadurch gekennzeichnet, dass** das erste Volumen (30) mit einer Querschnittsfläche, die weiter ist als eine entsprechende Querschnittsfläche eines von dem ersten Lumen (21) und dem zweiten Lumen (24), ausgelegt ist, um sich zwischen dem ersten Lumen (21) und dem zweiten Lumen (24) zu lokalisieren, wobei der Fluidstrom durch dieses erste Volumen zugelassen wird.

7. Fluidverbindung nach Anspruch 1, **dadurch gekennzeichnet, dass** der Hohlraum (29) ausgelegt ist, um einen Teil eines verbleibenden von dem ersten Körper (20) und dem zweiten Körper (23) zu bilden, der ohne den Vorsprung (28) ist.

8. Fluidverbindung nach Anspruch 1, **dadurch gekennzeichnet, dass** das zweite Volumen (31) ausgelegt ist, um sich hauptsächlich zwischen dem Vorsprung (28) und dem zweiten Körper (23) zu lokalisieren.

9. Fluidverbindung nach Anspruch 1, **dadurch gekennzeichnet, dass** zumindest einer von dem ersten Verbinder (19) und dem zweiten Verbinder (22) funktionell mit einer Fluidprobenentnahmeleitung (8) verbunden ist.

10. Fluidverbindung nach Anspruch 1, **dadurch gekennzeichnet, dass** der erste Verbinder (19) und der zweite Verbinder (22) verwendet werden, um einen Luftwegadapter funktionell mit einem von einem Intubationstubus (3) und einem Beatmungsgerät (2) zu verbinden.

11. Fluidverbindung nach Anspruch 1, **dadurch gekennzeichnet, dass** zumindest einer von dem ersten Verbinder (19) und dem zweiten Verbinder (22) funktionell mit einem Luftwegadapter (7) verbunden ist.

12. Fluidverbindung nach Anspruch 1, **dadurch gekennzeichnet, dass** der Durchmesser des zweiten Volumens einschließlich des Durchmessers des Vorsprungs (28) länger als der Durchmesser des ersten Volumens ist.

13. Fluidverbindung nach Anspruch 1, **dadurch gekennzeichnet, dass** eine Querschnittsfläche des zweiten Volumens (31) einschließlich des Vorsprungs (28) weiter als eine Querschnittsfläche des ersten Volumens (30) ist.

14. Fluidverbindung nach Anspruch 13, **dadurch gekennzeichnet, dass** die Querschnittsfläche als perpendikular zu einer Hauptrichtung des Stroms in dem ersten und zweiten Lumen gilt.

15. Fluidverbindung nach Anspruch 1, **dadurch gekennzeichnet, dass** der erste Verbinder (19) und der zweite Verbinder (22) voneinander lösbar sind.

## Revendications

1. Liaison fluidique destinée à réduire un volume de fluide dans ladite liaison comprenant :
un premier raccord (19) présentant un premier corps (20) entourant un premier alésage (21) permettant un écoulement de fluide le long dudit premier alésage, ledit premier corps présentant une première surface d'étanchéité (25), lequel premier alésage étant raccordé de manière opérationnelle à une ligne de prélèvement (8) présentant un diamètre interne (D1) ou à un tube d'intubation (3) présentant un diamètre interne (D5) ;
un second raccord (22) présentant un second corps (23) entourant un second alésage (24) permettant un écoulement de fluide le long dudit second alésage, ledit second corps présentant une seconde surface d'étanchéité (26) pouvant former une liaison étanche avec ladite première surface d'étanchéité (25) lorsque, à la fois, ledit premier raccord (19) et ledit second raccord (22) sont conjugués ;
une saillie (28) sur l'un dudit premier corps (20) entourant ledit premier alésage (21) et dudit second corps (23) entourant ledit second l'alésage (24), laquelle saillie entourant l'un dudit premier alésage et dudit second alésage,
un volume (17) entre ledit premier raccord (19) et ledit second raccord (22) lorsqu'ils sont conjugués, et
une cavité (29) dans l'un restant dudit premier corps et dudit second corps, laquelle cavité étant configurée de manière à recevoir ladite saillie lorsque ledit premier raccord et ledit second raccord sont conjugués, séparant ledit volume (17) en un premier volume (30) et un second volume (31) qui est à une certaine distance dudit premier volume,
**caractérisée en ce que** lesdits premier et second diamètres de l'alésage sont sensiblement égaux ou identiques au diamètre (D1) de ladite ligne de prélèvement (8) ou audit diamètre (D5) dudit tube d'intubation.

2. Liaison fluidique selon la revendication 1, **caractérisée en ce qu'**au moins l'un dudit premier corps (20) avec ladite première surface d'étanchéité (25) et dudit second corps (23) avec ladite seconde surface d'étanchéité (26) est équipé d'une surface conique formant une liaison étanche au fluide entre ladite première surface d'étanchéité et ladite seconde surface d'étanchéité lorsqu'elles sont conjuguées.

3. Liaison fluidique selon la revendication 1, **caractérisée en ce que**, à la fois, ladite première surface d'étanchéité (25) et ladite seconde surface d'étanchéité (26) sont coniques rendant possible une surface d'étanchéité suffisamment grande entre ledit premier raccord (19) et ledit second raccord (22) lorsqu'ils sont conjugués, auquel cas ledit premier corps (20) et ledit second corps (23) sont au moins partiellement l'un à l'intérieur de l'autre.

4. Liaison fluidique selon la revendication 1, caractérisée en ce ledit second volume (31) est configuré de manière à entourer ladite saillie (28) lorsque ledit premier corps (20) et ledit second corps (23) sont conjugués.

5. Liaison fluidique selon la revendication 1, **caractérisée en ce que** ledit premier volume (30) et ledit second volume (31) sont configurés de manière à permettre un échange mineur de gaz l'un avec l'autre au moyen d'un canal d'échange (32) entre ladite saillie (28) et ladite cavité (29) lorsque ledit premier raccord (19) et ledit second raccord (22) sont conjugués.

6. Liaison fluidique selon la revendication 1, **caractérisée en ce que** ledit premier volume (30) avec une section transversale plus grande qu'une section transversale correspondante de l'un dudit premier alésage (21) et dudit second alésage (24) est configuré afin de se positionner entre ledit premier alésage (21) et ledit second alésage (24), permettant le passage de fluide à travers ce premier volume.

7. Liaison fluidique selon la revendication 1, caractérisée en que ladite cavité (29) est configurée de manière à former une partie de l'un restant dudit premier corps (20) et dudit second corps (23) sans être associée à ladite saillie (28).

8. Liaison fluidique selon la revendication 1, **caractérisée en ce que** ledit second volume (31) est configuré de manière à se situer principalement entre ladite saillie (28) et ledit second corps (23),

9. Liaison fluidique selon la revendication 1, **caractérisée en ce qu'**au moins l'un dudit premier raccord (19) et dudit second raccord (22) est raccordé de manière opérationnelle à une ligne de prélèvement de fluide (8).

10. Liaison fluidique selon la revendication 1, **caractérisée en ce que** ledit premier raccord (19) et ledit second raccord (22) sont utilisés afin de raccorder de manière opérationnelle un adaptateur pour voies aériennes à l'un d'un tube d'intubation (3) et d'un ventilateur (2).

11. Liaison fluidique selon la revendication 1, **caractérisée en ce qu'**au moins l'un dudit premier raccord (19) et dudit second raccord (22) est raccordé de manière opérationnelle à un adaptateur pour voies aériennes (7).

12. Liaison fluidique selon la revendication 1, **caractérisée en ce que** le diamètre dudit second volume comportant le diamètre de ladite saillie (28) est supérieur au diamètre dudit premier volume.

13. Liaison fluidique selon la revendication 1, **caractérisée en ce qu'**une section transversale dudit second volume (31) comportant ladite saillie (28) est plus grande qu'une section transversale dudit premier volume (30).

14. Liaison fiuidique selon la revendication 13, **caractérisée en ce que** ladite section transversale est considérée être perpendiculaire à une direction principale de l'écoulement dans lesdits premier et second alésages.

15. Liaison fluidique selon la revendication 1, **caractérisée en ce que** ledit premier raccord (19) et ledit second raccord (22) peuvent être séparés l'un de l'autre.
